# EUROPEAN PATENT SPECIFICATION

(11) **EP 1 049 722 B1**
(45) Date of publication and mention of the grant of the patent: **17.04.2002**
(21) Application number: 99900881.6
(22) Date of filing: 18.01.1999
(51) Int. Cl.: C08B 37/06, C08L 5/06, A23L 1/0524

(54) **PECTIN FOR USE IN PASTE-LIKE MATERIALS, A METHOD OF PREPARING THE SAME, PASTE-LIKE MATERIALS COMPRISING THE PECTIN AS WELL AS THE USE THEREOF**
PEKTIN ZUR VERWENDUNG IN PASTÖSEN MATERIALIEN,VERFAHREN ZU DESSEN HERSTELLUNG,PASTÖSE MATERIALIEN, DIE DIESES PEKTIN ENTHALTEN UND DEREN VERWENDUNG
PECTINE UTILISEE DANS DES MATERIAUX PATEUX, SON PROCEDE DE PREPARATION, MATERIAUX PATEUX LA CONTENANT AINSI QUE LEUR UTILISATION

(30) Priority: 20.01.1998 DK 6998
(43) Date of publication of application: 08.11.2000
(73) Proprietor: CP Kelco ApS, 4623 Lille Skensved (DK)
(72) Inventor: MARR, Beinta, Unni, DK-2920 Charlottenlund (DK); CHRISTENSEN, Steen, Hojgaard, DK-2700 Bronshoj (DK)
(74) Representative: Klinge, Ulla Callesen
(86) International application number: DK9900026
(87) International publication number: WO9937685

(56) References cited:
- EP-A2- 0 580 252
- WO-A1-89/12648
- WO-A1-97/30093
- GB-A- 2 145 103
- GB-A- 2 311 024
- STN INTERNATIONAL, File CAPLUS, CAPLUS Accession No. 1984:607674, Document No. 101:207674, AIMUKHAMEDOVA G.B. et al., "Pectic Substances from Sea Buckthorn"; & OBLEPIKHA KRUSHINOVIDNAYA, 1983, 30-2 (Russ).
- STN INTERNATIONAL, File CAPLUS, CAPLUS Accession No. 1984:607674, Document No. 101:207674, AIMUKHAMEDOVA G.B. et al., "Pectic Substances from Sea Buckthorn";

## Description

### Technical Field

The invention relates to a pectin for use in paste-like materials, paste-like materials comprising the pectin as well as the use thereof.

### Background Art

Pectins can be used for the production of a number of foodstuffs, such as jam, jelly and fillings for pies etc., including heat tolerating such as in form of baking at up to approximately 200 to 250°C for up to 20 minutes without "flowing".

Industrially produced pectins are made up primarily of polygalacturonic acid chains in which rhamnose may be found. In addition, neutral sugars may be attached to the rhamnose units. The anhydrogalacturonic acid makes up at least 65% of the solids in commercial pectins. The galacturonic acids are partly esterified with methyl alcohol.

According to convention, pectins with more than 50% of the carboxylic acid groups esterified with methyl alcohol are referred to as high methoxyl pectins, whereas pectins with less than 50% of the carboxylic acid groups esterified with methyl alcohol are called low methoxyl pectins.

In the present description the expression "degree of esterification" means the degree in which the free carboxylic acid groups in the polygalacturonic acid chain have been esterified by way of methylation. In other words, the degree of esterification is defined as the number of esterified galacturonic acid units expressed in percentages of all the galacturonic acid units in the molecule, and accordingly it assumes a value between 0 and 100%.

The pectins hitherto used for heat stable foodstuffs comprise both pectins with a high degree of esterification and pectins with a low degree of esterification. Pectins with a high degree of esterification (DE) are characterised by resulting in a very strong and hard texture requiring a mechanical processing (a vigorous stirring) before they can be pumped or spread. The mechanical processing has, however, the effect that these pectins with a high DE cause a separation of water (syneresis) and consequently a reduced bake stability. Pectins with a low degree of esterification are characterised by being pumpable and spreadable, but in order to make them heat stable it is necessary to reduce the degree of esterification to below approximately 10 with the result that difficulties arise in connection with the dissolving of the pectin in hard water with degrees of hardness exceeding approximately 10°H (71.5 ppm calcium), and that a partial dissolving in distilled water is necessary.

WO-A-97/30093 discloses pectin fibres, which may be used in e.g. wound dressings and the like. The pectins disclosed therein have a DE of less than 15 % and a molecular weight in the range 5,000-200,000.

### Brief Description of the Invention

It turned out surprisingly that it is rendered possible by the use of a novel pectin with a molecular weight in the range of approximately 20,000 to 50,000 Daltons and a degree of esterification of less than approximately 20, preferably less than approximately 10, especially less than approximately 5, as the sole gelling agent or in combination with a pectin with a higher molecular weight and a degree of esterification of less than approximately 20, preferably less than approximately 10, and especially less than approximately 5, to achieve products which simultaneously are both pasty and heat stable. In addition the advantages are obtained that it is possible to use ordinary tap water for dissolving the pectin, and that a pasty texture is achieved without involving a vigorous stirring.

In the present description the expression "gelling agent" refers to an agent characterised by resulting in a structure being either solid or viscous.

When a combination of a low-molecular pectin and a high-molecular pectin is used, it is possible to tailor the pectin combination by varying the ratio of the low-molecular pectin, viz. the so-called primary pectin, to the high--molecular pectin, viz. the so-called secondary pectin, such that said combination has an optimum effect within various fields of application, such as in bake-stable jams with a high solids content and in pH-neutral "fillings" with a high solids content.

Therefore a first object of the invention is a pectin being characterised by having a molecular weight in the range of from approximately 20,000 to approximately 50,000 Daltons and a degree of esterification (DE) of less than approximately 20.

Another object of the invention is a method of producing the pectin according to the invention, said method being characterised by extracting the pectin in a manner known per se followed by said pectin being subjected to molecular weight reduction as well as a reduction of the degree of esterification.

The pectin according to the invention can be produced from any conventional pectin raw material, such as from grape-fruit, lemon, lime or orange peel, from which the pectin is extracted in a conventional manner. A typical method involves the steps:
1) acid extraction from the plant starting material at a low pH value,
2) purification of the liquid extract, and
3) isolation of the extracted pectin from the liquid.

In this connection reference is made to EP publication No. 664,300 A1 disclosing methods of producing pectin.

Thereafter the pectin obtained is subjected to an additional treatment in order to reduce the molecular weight from approximately 60,000 to 160,000 Daltons, preferably from approximately 80,000 to 120,000, to the desired range of approximately 20,000 to 50,000 Daltons, preferably approximately 25,000 to 40,000 Daltons, and in order to reduce the degree of esterification from approximately 55-75%, preferably from 55-65%, to less than approximately 20%, preferably less than approximately 10%, and especially less than approximately 5%.

The reduction of the degree of esterification can be performed by way of alkaline treatment, such as by means of an alkali metal hydroxide, such as NaOH, KOH or LiOH, or by means of an alkaline earth metal hydroxide, such as Ca(OH)₂ or Mg(OH)₂. The reduction of the degree of esterification can also be performed enzymatically, optionally by means of pectin methyl esterase. Moreover, the degree of esterification can be reduced by combining an acid treatment by means of an inorganic acid, such as HNO₃, HCl or H₂SO₄, preferably HNO₃, with an alkaline treatment and/or an enzymatic treatment.The degree of esterification is titrimetrically determined, for instance by means of the FCC and FAO/WHO method, cf. Food Chemicals Codex, FCC IV Monographs, 4th. ed., National Academy Press, Washington DC, 283 (1996) as well as FAO, Food and Nutrition Paper 52. Addendum 1, Rome, 87 (1992).

The molecular weight reduction can be performed enzymatically or by means of alkali. When the molecular weight reduction is performed by means of an alkali, an alkali metal hydroxide, such as NaOH, KOH or LiOH, preferably NaOH, or an alkaline earth metal hydroxide, such as Ca(OH)₂ or Mg(OH)₂ is preferably used. The reduction of the molecular weight can also be performed enzymatically by means of for instance endopolygalacturonase or pectin lyase or alternative pectate lyase. When pectate lyase is used, it is assumed that the degree of esterification has been reduced prior to the pectate lyase treatment.

The molecular weight can be determined by way of glass capillary viscosimetry, whereby the relative viscosity of an 0.1% pectin solution is measured by means of Na-hexametaphosphate, jf. Christensen, P.E.: "-Methods of Grading Pectin in Relation to the Molecular Weight (Intrinsic Viscosity) of Pectin", Food Research, Volume 19, pages 163 through 171 (1954).

The molecular weight reduction as well as the reduction of the degree of esterification can be performed in one or more steps. When a two-step process is followed, it is often chosen to reduce the degree of esterification first followed by the molecular weight reduction, but the sequence is not decisive.

The reduction of the degree of esterification and/or the molecular weight can be performed between steps 2 and 3 in the process for obtaining pectin as described above, cf. EP publication No. 664,300 A1, and/or after step 3.

The pectin according to the invention can be used either alone or in combination with a high-molecular pectin for the production of paste-like materials.

Therefore the invention also relates to a paste-like material comprising as gelling agent a pectin according to the invention.

According to a preferred embodiment of the invention, the paste-like material comprises a combination of least one pectin according to the invention, viz. the primary pectin, and at least one secondary pectin with a molecular weight in the range of approximately 50,000 to 150,000 Daltons and a degree of esterification of less than approximately 20. The secondary pectin has preferably a degree of esterification of less than approximately 10, especially of less than approximately 5. In addition, the secondary pectin has preferably a molecular weight in the range of 60,000 to 110.000 Daltons, especially approximately 80,000 to 90,000. An example of a secondary pectin is GENU Pectin type LM-5 CS, produced by Copenhagen Pectin A/S.

When a combination of a primary pectin and a secondary pectin is used, the ratio of the primary pectin to the secondary pectin is in the range of from approximately 5:1 to approximately 1:3, preferably from approximately 3:1 to approximately 1:1.

The use of a combination of a primary pectin and a secondary pectin renders it possible by way of a control of the mutual proportional ratio to tailor the paste-like material to a specific use, such as for bake stable jams with a high solids content and produced on the basis of any desired fruit, such as strawberry, raspberry, apple, apricot or the like fruit.

According to another object the invention therefore relates to the use of a paste-like material in a fruit-based product, preferably in an amount in the range of 0.8 to 1.5% by weight, especially approximately 1.0 to 1.2% by weight.

Furthermore, the paste-like material can be used for neutral fillings, such as milk-based creams with for instance vanilla or caramel taste, preferably in an amount in the range of 0.8 to 1.5% by weight, especially approximately 1.0 to 1.2% by weight.

In addition to the above fields of application the invention can also be used for other types of foodstuffs desired to be gelled, such as vegetable pastes, for instance tomato paste, or meat or fish-based products, such as pâté.

The ratio of the primary to the secondary pectin varies generally in response to the solids content in the final product. Thus, high solids contents (SS = soluble solids) of about 75% involve the use of a relatively high proportion of primary pectin, whereas lower solids contents involve the use of a comparatively lower proportion of primary pectin.

Here it should be noted that as a (partial) replacement or supplement of the secondary pectin it is possible to vary the Ca²⁺-content in the final product so as to adjust the product characteristics. The latter is carried out by adding an amount of food quality calcium salt corresponding to a calcium amount of 0 to 500 ppm Ca²⁺, preferably approximately 50 to 200 ppm Ca²⁺, taking into account the natural calcium-content of the foodstuff in question.

The invention is illustrated below by means of the following Examples.

### EXAMPLES

### Example 1

### Preparation of primary pectin.

### a) Molecular weight reduction

4.035 kg of liquid extract of citrus peels comprising 36.6 g of dry pectin with a molecular weight of approximately 100,000 and a degree of esterification DE of approximately 62 are introduced in a tube reactor and adjusted at a pH value of 4.0 by addition of Na₂CO₃. The input temperature is set at 47°C ± 2°C, whereafter a solution of endopolygalacturonase sold under the trade name Rohament P by the company Röhm is added in a concentration of 75 g of enzyme/100 I solution. The enzymatic treatment is carried out for approximately 15 minutes, whereafter the enzyme is deactivated at 70 to 75°C for approximately 10 minutes.

After the enzyme activation, the juice is subjected to an evaporation until it has reached approximately one sixth of the starting amount. The pH value is adjusted to 2.0 by means of HNO₃, and the juice is subjected to a pressure filtration on Celite® 545.

The pressure filtered juice is cooled to approximately 20 to 30°C, and the enzymatically treated pectin is precipitated with 1 part of juice to 2 parts of 80% isopropanol (IPA). Then the pectin is processed by passing through a drum screen, whereafter the retentate is carried through a band press.

The molecular weight of the pectin after the enzymatic treatment: 33,000 Daltons.

### b) Deesterification

The deesterification in a tank reactor uses NaOH in 60% isopropanole at a temperature of 5°C, the NaOH-concentration being 0.3 eq/I.

The pectin material produced above is treated with alkali for approximately 1 to 2 hours, whereafter a postwashing is carried out with pure 60% isopropanol. Then the material is carried to a band press, and the filtrate is stripped for alcohol in a Lödige-mixer and dried in a drying chamber.

The resulting pectin has a degree of esterification of 4.6%.

### Example 2

1 kg of dry pectin with a molecular weight of approximately 100,000 and a degree of esterification of approximately 70% is suspended in 5.7 I of 55% isopropanol comprising 380 ml of 28% NaOH in a tank reactor. The temperature is adjusted at 60°C and kept there. After 1 hour the suspension is removed and filtered through a cloth, and the extracted solid matter is washed in 2 times 10 I of 60% isopropanol. Subsequently, the pectin is suspended in 10 I of 60% isopropanol, and the pH value is adjusted to 4.5 to 5.0 by means of 84% phosphoric acid. The pectin is washed again in 10 I of 60% isopropanol and dried.

A pectin with the following characteristics is obtained:
Molecular weight: 35,000 Daltons
DE: 5.2%

### Example 3

9 kg of dry pectin with a molecular weight of approximately 110,000 and a degree of esterification of approximately 69% is suspended while stirred in 51 I of 55% IPA admixed 4.2 I of 28% NaOH, and the temperature is adjusted at 45°C.

After 1 hour at this temperature, the pectin is extracted in the same manner as in Example 2.

A pectin with the following characteristics is obtained:
Molecular weight: 28,000 daltons
DE: 1.7%

### Example 4

### a) Deesterification

22.4 kg of wet pectin obtained in a conventional manner as described in the above EP 664,300 A1, with a solids content of 25% and with a molecular weight of approximately 90,000 and a degree of esterification of approximately 60% are suspended in 320 I of 55% IPA admixed 2.4 I of 28% NaOH at at temperature of 5°C for 2 hours in a tank reactor.

Degree of esterification of the pectin after alkali treatment: 3 to 5% Molecular weight of the pectin after alkali treatment: 70,000

### b) Molecular weight reduction

2.8 kg of dry pectin from step a) are suspended in 16 I of 55% IPA admixed 190 ml of 28% NaOH at a temperature of 60°C in a tank reactor.

The suspension is removed after 2 hours and processed as described in Example 2.

A pectin with the following characteristics is obtained:
Molecular weight: 38,000 Daltons
DE: 2.0%

### Example 5

A pectin product is produced which is pumpable and can be dosed, and which can be dissolved in ordinary tap water with a hardness of up to 10°H by way of a simple mixing of the ingredients.

### Composition.

| Ingredients | Amount % |
|---|---|
| pectin according to Example 1 | 70.0% |
| icing sugar | 30.0% |

### Example 6

A pectin product is produced which is pumpable and can be dosed, and which can be dissolved in ordinary tap water with a hardness of up to 10°H by way of a simple mixing of the ingredients.

### Composition:

| Ingredients | Amount, % |
|---|---|
| pectin according to Example 1 | 52.5% |
| GENU Pectin type LM-5 CS* | 17.5% |
| icing sugar | 30.0% |

| | |
|---|---|
| * Produced by Copenhagen Pectin A/S | |

### Example 7

Analogy to Example 6, a pectin product is produced which is pumpable and can be dosed, and which can be dissolved in water with a hardness of 10°H or more, and which requires no chopping equipment during the production.

### Composition:

| Ingredients | Amount% |
|---|---|
| pectin according to Example 2 | 52.5% |
| GENU Pectin type LM-5 CS | 17.5% |
| icing sugar | 30.0% |

### Example 8

Analogy to Example 6, a pectin product is produced which is pumpable and can be dosed, and which can be dissolved in water with a hardness of 10°H or more.

### Composition:

| Ingredients | Amount % |
|---|---|
| pectin according to Example 3 | 35.0% |
| GENU pectin type LM-5 CS | 35.0% |
| icing sugar | 30.0% |

### Example 9

Like in Example 6, a pectin product is produced which is pumpable and can be dosed, and which can be dissolved in water with a hardness of 10°H or more.

### Composition:

| Ingredients | Amount, % |
|---|---|
| pectin according to Example 4 | 35.0% |
| GENU pectin type LM-5 CS | 35.0% |
| icing sugar | 30.0% |

### EXAMPLES OF APPLICATION

The following Examples illustrate the use of the above pectin products for the production of bake stable products.

### EXAMPLE OF APPLICATION 1

A bake stable jam is produced according to the following recipe.

| Order of addition | Ingredients | kg or litres | % of soluble solids (SS) | kg SS |
|---|---|---|---|---|
| A | Strawberry sauce | 20.0 | 10 | 2.0 |
| | Saccharose | 53.0 | 100 | 53.0 |
| | Syrup with a high fructose content | 27.6 | 70 | 19.3 |
| | | | | |
| B | Pectin product acc. Example 5 | 1.2 | 100 | 1.2 |
| | Water | 11.0 | - | - |
| | | | | |
| C | Citric acid, 50 weight/volume% solution | 0.7 | 50 | 0.35 |
| | Total ingredients | 113.5 | | 75.85 |
| | Evaporation | 13.5 | | |
| | Yield | 100.0 | | 75.9 |

- pH value in product :: 3.5 to 3.6
- Water activity a_{w} :: 0.70

The production of the bake stable jam is carried out by a method comprising the following steps:
1) the ingredients (A) are mixed,
2) a heating to the boiling point is performed and a boiling is carried out until the content of soluble solids (SS) is 80%,
3) the pectin product according to Example 5 is dissolved in hot water (B) by means of a Silverson L4R-mixer with a speed of 6,000 to 8,000 rpm
4) the pectin solution is added to the batch,
5) the content of soluble solids is adjusted to 75% by means of a manual refractometer (45 to 80% of soluble solids),
6) citric acid (C) is added in order to adjust the pH value to 3.5 to 3.6 and
7) a cooling to 70°C is carried out followed by a filling of the product in glasses.

The produced strawberry jam is tested with respect to bake stability by baking for 10 minutes at 200°C. The product turned out to have a satisfactory bake stability since it does not flow.

The testing of the bake stability involves the following test.

A portion of jam of 10 g is placed on filter paper in a metal ring of well-defined dimensions (height 7 mm, diameter 35 mm). Before the placing in an oven, the ring is removed, and after 10 minutes at 200°C, a sample is taken. As a measure of the bake stability, an inner annular diameter is indicated on the filter paper. By an optimum bake stability the jam keeps within this ring.

In addition, the surface must appear smooth. According to an alternative baking test, the jam is placed in the middle of a puff paste slice and baked for 20 minutes at 220°C. The bake stability is visually evaluated.

### EXAMPLE OF APPLICATION II

| Order of addition | Ingredients | kg or litres | % of souble solids (SS) | kg SS |
|---|---|---|---|---|
| A | Strawberry sauce | 20.0 | 10 | 2.0 |
| | Saccharose | 53.0 | 100 | 53.0 |
| | Syrup with a high fructose content | 27.6 | 70 | 19.3 |
| | | | | |
| B | Pectin product acc. to Example 6 | 1.2 | 100 | 1.2 |
| | Water | 11.0 | - | - |
| | | | | |
| C | Citric acid, 50 weight/volume% solution | 0.7 | 50 | 0.35 |
| | Total ingredients | 113.5 | | 75.85 |
| | Evaporation | 13.5 | | |
| | Yield | 100.0 | | 75.9 |

- pH value in product:: 3.5 to 3.6
- Water activity a_{w} :: 0.70

The jam is produced in the same manner as in example of application I.

The produced strawberry jam is tested with respect to bake stability by baking for 10 minutes at 200°C. The product reveals a good bake stability, and the low water activity ensures a long life and prevents the baked biscuits from turning soft.

### EXAMPLE OF APPLICATION III

A bake stable jam is produced according to the following recipe.

| Order of addition | Ingredients | kg or litres | % of soluble solids (SS) | kg SS |
|---|---|---|---|---|
| A | Strawberry sauce | 20.0 | 10 | 2.0 |
| | Saccharose | 53.0 | 100 | 53.0 |
| | Syrup with a high fructose content | 27.6 | 70 | 19.3 |
| B | Pectin product acc. to Example 7 | 1.2 | 100 | 1.2 |
| | Water | 11.0 | - | - |
| C | Citric acid, 50 weight/volume% solution | 0.7 | 50 | 0.35 |
| | Total ingredients | 113.5 | | 75.85 |
| | Evaporation | 13.5 | | |
| | Yield | 100.0 | | 76 |

- pH value in product :: 3.5 to 3.6
- Water activity a_{w} :: 0.70

The jam is produced in the same manner as in example of application I.

The produced strawberry jam is tested with respect to bake stability by baking for 10 minutes at 200°C. The product reveals a good bake stability, and the low water activity ensures a long life and prevents the baked biscuits from turning soft.

### EXAMPLE OF APPLICATION IV

A bake stable jam is produced according to the following recipe.

| Order of addition | Ingredients | kg or litres | % of soluble solids (SS) | kg SS |
|---|---|---|---|---|
| A | Strawberry sauce | 20.0 | 10 | 2.0 |
| | Saccharose | 48.7 | 100 | 48.7 |
| | Syrup with a high fructose content | 25.3 | 70 | 17.7 |
| | | | | |
| B | Pectin product acc. to Example 8 | 1.2 | 100 | 1.2 |
| | Water | 11.0 | - | - |
| | | | | |
| C | Citric acid, 50 weight/volume % solution | 0.7 | 50 | 0.35 |
| | Total ingredients | 106.9 | | 70.0 |
| | Evaporation | 6.9 | | |
| | Yield | 100.0 | | 70.0 |

- pH value in product :: 3.5 to 3.6
- water activity a_{w} :: 0.75

The jam is produced in the same manner as in Example of Application I, whereby, however, in step 2) the solids content is adjusted to approximately 75% and in step 5) the solids content is adjusted to 70%.

The product is tested with respect to bake stability and reveals a good bake stability on filter paper at 200°C for 10 minutes.

### EXAMPLE OF APPLICATION V

A bake stable jam is produced according to the following recipe.

| Order of addition | Ingredients | kg or litres | % of soluble solids (SS) | kg SS |
|---|---|---|---|---|
| A | Strawberry sauce | 20.0 | 10 | 2.0 |
| | Saccharose | 45.1 | 100 | 45.1 |
| | Syrup with a high fructose content | 23.4 | 70 | 16.4 |
| | | | | |
| B | Pectin product acc. to Example 9 | 1.1 | 100 | 1.1 |
| | Water | 20.0 | - | - |
| | | | | |
| C | Citric acid, 50 weight/volume % solution | 0.7 | 50 | 0.35 |
| | Total ingredients | 110.3 | | 65.0 |
| | Evaporation | 10.4 | | |
| | Yield | 100.0 | | 65.1 |

- pH value in product :: 3.5 to 3.7
- Water activity a_{w} :: 0.82

The jam is produced analogously with the method stated in Example of Application I, whereby, however, in step 2) the solids content is adjusted to approximately 70% SS and in step 5) the solids content is adjusted to 65% SS.

The product is bake stable on filter paper at 200°C for 10 minutes.

### EXAMPLE OF APPLICATION VI

A bake stable jam is produced according to the following recipe.

| Order of addition | Ingredients | kg or litres | % of soluble solids (SS) | kg SS |
|---|---|---|---|---|
| A | Strawberry sauce | 5.0 | 10 | 0.5 |
| | Saccharose | 53.6 | 100 | 53.6 |
| | Syrup with a high fructose content | 27.9 | 70 | 19.5 |
| | | | | |
| | Deionized water | 14.1 | | |
| B | Pectin product acc. to Example 8 | 1.0 | 100 | 1.0 |
| | Water | 11.0 | - | - |
| | | | | |
| C | Citric acid, 50 weight/volume% solution | 1.3 | 50 | 0.65 |
| | Total ingredients | 113.9 | | 75.3 |
| | Evaporation | 13.9 | | |
| | Yield | 100.0 | | 70 |

- pH value in product :: 3.5 to 3.6
- Water activity a_{w} :: 0.75

The jam is produced in the same manner as in example of application I.

The product is tested with respect to bake stability and reveals a good bake stability on filter paper at 200°C for 10 minutes.

### EXAMPLE OF APPLICATION VII

A bake stable jam is produced according to the following recipe.

| Order of addition | Ingredients | kg or litres | % of soluble solids (SS) | kg SS |
|---|---|---|---|---|
| A | Strawberry sauce | 30.0 | 10 | 3.0 |
| | Saccharose | 51.7 | 100 | 51.7 |
| | Syrup with a high fructose content | 26.9 | 70 | 18.8 |
| | | | | |
| B | Pectin product acc. to Example 8 | 1.2 | 100 | 1.2 |
| | Water | 11.0 | - | - |
| | | | | |
| C | Citric acid, 50 weight/volume% solution | 0.7 | 50 | 0.35 |
| | Total ingredients | 121.5 | | 75.0 |
| | Evaporation | 21.5 | | |
| | Yield | 100.0 | | 70.0 |

- pH value in product :: 3.5 to 3.6
- water activity a_{w} :: 0.75

The jam is produced in the same manner as in example of application I.

The product is tested with respect to bake stability and reveals a good bake stability on filter paper at 200°C for 10 minutes.

### EXAMPLE OF APPLICATION VIII

A bake stable jam is produced according to the following recipe.

| Order of addition | Ingredients | kg or litres | % of soluble solids (SS) | kg SS |
|---|---|---|---|---|
| A | Raspberry sauce | 20.0 | 10 | 2.0 |
| | Saccharose | 48.7 | 100 | 48.7 |
| | Syrup with a high fructose content | 25.3 | 70 | 17.7 |
| | | | | |
| B | Pectin product acc. to Example 8 | 1.2 | 100 | 1.2 |
| | Water | 11.0 | - | - |
| | | | | |
| C | Citric acid, 50 weight/volume% solution | 0.7 | 50 | 0.35 |
| | Total ingredients | 106.9 | | 70.0 |
| | Evaporation | 6.9 | | |
| | Yield | 100.0 | | 70.0 |

- pH value in product :: 3.5 to 3.6
- Water activity a_{w} :: 0.75

The jam is produced in the same manner as in Example of Application I, whereby, however, in step 2) the solids content is adjusted to approximately 75% and in step 5) the solids content is adjusted to approximately 70%.

The product is tested with respect to bake stability and reveals a good bake stability on filter paper at 200°C for 10 minutes.

### EXAMPLE OF APPLICATION IX

A bake stable hazelnut filling is produced according to the following recipe

| Order of addition | Ingredients | kg or litres | % of soluble solids (SS) | kg SS |
|---|---|---|---|---|
| A | Pectin product acc. to Example 6 | 0.5 | 100 | 0.5 |
| | Hot water | 14.0 | - | - |
| | | | | |
| B | Syrup with a high fructose content | 57.0 | 70 | 40 |
| | | | | |
| C | Skimmed milk powder | 8.0 | 100 | 0.8 |
| | Fat-reduced cocoa | 6.0 | 100 | 6.0 |
| | Vanilla flavour | 0.3 | 100 | 0.3 |
| | Dry cream flavour | 0.3 | 100 | 0.3 |
| | Hazelnut paste | 14.0 | 100 | 14.0 |
| | | | | |
| D | | | | |
| | Total ingredients | 100.1 | | 69.1 |

- pH value in product:: 6.0
- Water activity a_{w} :: 0.8

The production of the bake stable hazelnut filling is carried out by a method comprising the following steps:
1) The pectin product according to Example 6 is dissolved in hot water (the ingredients (A)) by means of a Silverson L4R-mixer with a speed of 6,000 to 8,000 rpm
2) ingredient (B) is added while subjected to a stirring,
3) the ingredients (C) are dry mixed and added to the solution, whereafter the mixing continues for 5 minutes,
4) the hazelnut paste is added during the mixing, and
5) a cooling to 70°C is effected, whereafter the product can be filled into for instance bread-like materials, such as croissants.

The produced hazelnut filling has the following properties:
- good bake stability *
- controlled texture
- good release of flavours
- flexible filling temperature
- reduced fat content.

* The bake stability is tested by placing the above product in the middle of a puff paste slice and baking it for 20 minutes at 220°C. The bake stability is visually evaluated.

### COMPARISON TEST 1

In the same manner as in Example 2, two pectins are produced with a molecular weight of 10,000 and 18,000, respectively, and a degree of esterification of approximately 10%.

The test of the products with respect to bake stability analogously with the Examples of Application I-VIII revealed that the products were light-fluid and caused syneresis, and that the filling texture was too thin. The baking test on filter paper revealed that the jam flowed 4 to 5 cm beyond the indicated annular diameter.

### COMPARISON TEST 2

In the same manner as in Example 2, two pectins are produced with a molecular weight of approximately 18,000 and a degree of esterification of 17% and 19%, respectively.

The low viscosity of the solutions resulted in a poor result for both solutions, the filling being light-fluid and causing syneresis at the bake stability test. The jam flowed 3 to 4 cm beyond the annular diameter.

### COMPARISON TEST 3

A pectin with a molecular weight of approximately 29,000 and a degree of esterification of approximately 24% was produced in the same manner as in Example 2.

The solution was low-viscous and provided an unsatisfactory result by the bake stability test, the filling becoming light-fluid and causing syneresis.

The jam flowed 3 to 4 cm beyond the annular diameter.

## Claims

1. A pectin for use in paste-like materials, **characterised by** having a molecular weight in the range of from > 25,000 to 50,000 Daltons and a degree of esterification (DE) of less than 20.

2. The pectin as claimed in claim 1, **characterised by** having a DE of less than 10, preferably less than 5.

3. The pectin as claimed in claim 1 or 2, **characterised by** having a DE of 1.7.

4. The pectin as claimed in claims 1 to 3, **characterised by** having a molecular weight in the range of from > 25,000 to 40,000 Daltons.

5. A method of producing the pectin as claimed in claims 1 to 4, **characterised by** subjecting a pectin starting material to a molecular weight reduction as well as a reduction of the degree of esterification.

6. The method as claimed in claim 5, **characterised by** the molecular weight reduction and the reduction of the degree of esterification being performed in one step.

7. The method as claimed in claim 5, **characterised by** the reduction of the degree of esterification and the molecular weight reduction being performed in two steps, preferably by initially reducing the degree of esterification followed by a reduction of the molecular weight.

8. The method as claimed in any of the claims 5 to 7, **characterised by** the reduction of the degree of esterification being performed by means of alkali, such as an alkali metal hydroxide, such as NaOH, KOH or LiOH, or by means of an alkaline earth metal hydroxide, such as Ca(OH)₂ or Mg(OH)₂, or enzymatically, such as by means of pectin methylesterase, or by means of a combination of an acid treatment with an inorganic acid, such as HNO₃, HCI or H₂SO₄, preferably HNO₃, with an alkaline treatment and/or an enzymatic treatment.

9. The method as claimed in any of the claims 5 to 7, **characterised by** the molecular weight reduction being performed enzymatically, such as by means of endopolygalacturonase, pectin lyase or pectate lyase, or by means of alkaline treatment, such as by means of an alkali metal hydroxide, especially NaOH, KOH or LiOH, preferably NaOH, or by means of an alkaline earth metal hydroxide, such as Ca(OH)₂ or Mg(OH)₂.

10. A paste-like material, **characterised in that** as gelling agent it comprises a pectin as claimed in any of the claims 1 to 4.

11. The paste-like material as claimed in claim 10, **characterised in that** as gelling agent it comprises a combination of at least one primary pectin as claimed in any of the claims 1 to 4 and at least one secondary pectin with a molecular weight in the range of from 50,000 to 150,000 Daltons and a degree of esterification of less than 20.

12. The paste-like material as claimed in claim 11, in which the secondary pectin has a DE of less than 10, preferably less than 5.

13. The paste-like material as claimed in claims 10 to 12, in which the secondary pectin has a molecular weight in the range of from 60,000 to 110,000 Daltons, preferably from 80,000 to 90,000 Daltons.

14. The paste-iike material as claimed in any of claims 11 to 13, in which the ratio of primary pectin to secondary pectin is in the range of from 5:1 to 1:3, prefarably in the range of from 3:1 to 1:1.

15. A use of a pectin as claimed in any of the claims 1 to 4 for the production of a paste-like material as claimed in any of the claims 10 to 14.

16. The use of a paste-like material as claimed in any of the claims 10 to 14 in a fruit-based product.

17. The use as claimed in claim 16, in which the paste-like material is used in an amount in the range of 0.8 to 1.5% by weight, preferably 1.0 to 1.2% by weight.

18. The use of a paste-like material as claimed in any of the claims 10 to 14 in a milk-based product.

19. The use as claimed in claim 18, in which the paste-like material is used in an amount in the range of 0.8 to 1.5 %by weight, preferably 1.0 to 1.2% by weight.

## Patentansprüche

1. Pektin zur Verwendung in pastenartigen Materialien, **dadurch gekennzeichnet, daß** es ein Molekulargewicht im Bereich von > 25.000 bis 50.000 Dalton und einen Veresterungsgrad (VG) von weniger als 20 aufweist.

2. Pektin nach Anspruch 1, **dadurch gekennzeichnet, daß** es einen VG von weniger als 10, vorzugsweise weniger als 5, aufweist.

3. Pektin nach Anspruch 1 oder 2, **dadurch gekennzeichnet, daß** es einen VG von 1,7 aufweist.

4. Pektin nach den Ansprüchen 1 bis 3, **dadurch gekennzeichnet, daß** es ein Molekulargewicht im Bereich von > 25.000 bis 40.000 Dalton aufweist.

5. Verfahren zur Herstellung des Pektins nach einem der Ansprüche 1 bis 4, **dadurch gekennzeichnet, daß** man ein Pektin-Edukt einer Molekulargewichtsverringerung sowie einer Verringerung des Veresterungsgrads unterwirft.

6. Verfahren nach Anspruch 5, **dadurch gekennzeichnet, daß** man die Molekulargewichtsverringerung und die Verringerung des Veresterungsgrads in einem Schritt durchführt.

7. Verfahren nach Anspruch 5, **dadurch gekennzeichnet, daß** man die Verringerung des Veresterungsgrads und die Molekulargewichtsverringerung in zwei Schritten durchführt, und zwar vorzugsweise dadurch, daß man zunächst den Veresterungsgrad und dann das Molekulargewicht verringert.

8. Verfahren nach einem der Ansprüche 5 bis 7, **dadurch gekennzeichnet, daß** man die Verringerung des Veresterungsgrads mit Alkali, wie einem Alkalimetallhydroxid, wie NaOH, KOH oder LiOH, oder mit einem Erdalkalimetallhydroxid, wie Ca(OH)₂ oder Mg(OH)₂, oder enzymatisch, wie mit Pektin-Methylesterase, oder mit Hilfe einer Kombination aus einer Säurebehandlung mit einer anorganischen Säure, wie HNO₃, HCl oder H₂SO₄, vorzugsweise HNO₃, und einer Alkalibehandlung und/oder einer enzymatischen Behandlung durchführt.

9. Verfahren nach einem der Ansprüche 5 bis 7, **dadurch gekennzeichnet, daß** man die Molekulargewichtsverringerung enzymatisch, wie mit Endopolygalacturonase, Pektinlyase oder Pektatlyase, oder mit Hilfe einer Alkalibehandlung, wie mit einem Alkalimetallhydroxid, insbesondere NaOH, KOH oder LiOH, vorzugeweise NaOH, oder mit einem Erdalkalimetallhydroxid, wie Ca(OH)₂ oder Mg(OH)₂, durchführt.

10. Pastenartiges Material, **dadurch gekennzeichnet, daß** es als Geliermittel ein Pektin nach einem der Ansprüche 1 bis 4 enthält.

11. Pastenartiges Material nach Anspruch 10, **dadurch gekennzeichnet, daß** es als Geliermittel eine Kombination aus mindestens einem primären Pektin nach einem der Ansprüche 1 bis 4 und mindestens einem sekundären Pektin mit einem Molekulargewicht im Bereich von 50.000 bis 150.000 Dalton und einem Veresterungsgrad von weniger als 20 enthält.

12. Pastenartiges Material nach Anspruch 11, bei dem das sekundäre Pektin einen VG von weniger als 10, vorzugsweise weniger als 5, aufweist.

13. Pastenartiges Material nach den Ansprüchen 10 bis 12, bei dem das sekundäre Pektin ein Molekulargewicht im Bereich von 60.000 bis 110.000 Dalton, vorzugsweise von 80.000 bis 90.000 Dalton, aufweist.

14. Pastenartiges Material nach einem der Ansprüche 11 bis 13, bei dem das Verhältnis von primärem Pektin zu sekundärem Pektin im Bereich von 5:1 bis 1:3, vorzusgweise im Bereich von 3:1 bis 1:1, liegt.

15. Verwendung eines Pektins nach einem der Ansprüche 1 bis 4 zur Herstellung eines pastenartigen Materials nach einem der Ansprüche 10 bis 14.

16. Verwendung eines pastenartigen Materials nach einem der Ansprüche 10 bis 14 in einem Produkt auf Fruchtbasis.

17. Verwendung nach Anspruch 16, bei der das pastenartige Material in einer Menge im Bereich von 0,8 bis 1,5 Gew.-%, vorzugsweise 1,0 bis 1,2 Gew.-%, verwendet wird.

18. Verwendung eines pastenartigen Materials nach einem der Ansprüche 10 bis 14 in einem Produkt auf Milchbasis.

19. Verwendung nach Anspruch 18, bei der das pastenartige Material in einer Menge im Bereich von 0,8 bis 1,5 Gew.-%, vorzugsweise 1,0 bis 1,2 Gew.-%, verwendet wird.

## Revendications

1. Pectine pour une utilisation dans des matières pâteuses, **caractérisée en ce qu'**elle a une masse moléculaire dans la plage de > 25 000 à 50 000 daltons et un degré d'estérification (DE) inférieur à 20.

2. Pectine selon la revendication 1, **caractérisée en ce qu'**elle a un DE inférieur à 10, de préférence inférieur à 5.

3. Pectine selon la revendication 1 ou 2, **caractérisée en ce qu'**elle a un DE de 1,7.

4. Pectine selon les revendications 1 à 3, **caractérisée en ce qu'**elle a une masse moléculaire dans la plage de > 25 000 à 40 000 daltons.

5. Méthode de production d'une pectine selon les revendications 1 à 4, **caractérisée en ce que** l'on soumet une matière première de pectine à une réduction de masse moléculaire ainsi qu'à une réduction de degré d'estérification.

6. Méthode selon la revendication 5, **caractérisée en ce que** la réduction de masse moléculaire et la réduction de degré d'estérification sont réalisées en une étape.

7. Méthode selon la revendication 5, **caractérisée en ce que** la réduction de degré d'estérification et la réduction de masse moléculaire sont réalisées en deux étapes, de préférence en réduisant d'abord le degré d'estérification, puis en réduisant la masse moléculaire.

8. Méthode selon l'une quelconque des revendications 5 à 7, **caractérisée en ce que** la réduction de degré d'estérification est réalisée au moyen d'une base, comme un hydroxyde de métal alcalin, tel que NaOH, KOH ou LiOH, ou au moyen d'un hydroxyde de métal alcalino-terreux, tel que Ca(OH)₂ ou Mg(OH)₂, ou par voie enzymatique, comme au moyen d'une pectine méthylestérase, ou au moyen d'une combinaison d'un traitement acide avec un acide minéral tel que HNO₃, HCl ou H₂SO₄, de préférence HNO₃, avec un traitement par une base et/ou un traitement enzymatique.

9. Méthode selon l'une quelconque des revendications 5 à 7, **caractérisée en ce que** la réduction de la masse moléculaire est réalisée par voie enzymatique comme au moyen d'une endopolygalacturonase, d'une pectine lyase ou d'une pectate lyase ou par traitement par une base, comme au moyen d'un hydroxyde de métal alcalin, en particulier NaOH, KOH ou LiOH, de préférence NaOH, ou au moyen d'un hydroxyde de métal alcalino-terreux, tel que Ca(OH)₂ ou Mg(OH)₂.

10. Matière pâteuse, **caractérisée en ce qu'**elle comprend, en tant qu'agent gélifiant, une pectine selon l'une quelconque des revendications 1 à 4.

11. Matière pâteuse selon la revendication 10, **caractérisée en ce qu'**elle comprend, en tant qu'agent gélifiant, une combinaison d'au moins une pectine primaire selon l'une quelconque des revendications 1 à 4 et au moins une pectine secondaire ayant une masse moléculaire dans la plage de 50 000 à 150 000 daltons et un degré d'estérification inférieur à 20.

12. Matière pâteuse selon la revendication 11, dans laquelle la pectine secondaire a un DE inférieur à 10, de préférence inférieur à 5.

13. Matière pâteuse selon les revendications 10 à 12, dans laquelle la pectine secondaire a une masse moléculaire dans la plage de 60 000 à 110 000 daltons, de préférence de 80 000 à 90 000 daltons.

14. Matière pâteuse selon l'une quelconque des revendications 11 à 13, dans laquelle le rapport de la pectine primaire à la pectine secondaire est dans la plage de 5 : 1 à 1 : 3, de préférence dans la plage de 3 : 1 à 1 : 1.

15. Utilisation d'une pectine selon l'une quelconque des revendications 1 à 4, pour la production d'une matière pâteuse selon l'une quelconque des revendications 10 à 14.

16. Utilisation d'une matière pâteuse selon l'une quelconque des revendications 10 à 14 dans un produit à base de fruits.

17. Utilisation selon la revendication 16, dans laquelle la matière pâteuse est utilisée en une quantité dans la plage de 0,8 à 1,5% en masse, de préférence de 1,0 à 1,2% en masse.

18. Utilisation d'une matière pâteuse selon l'une quelconque des revendications 10 à 14 dans un produit à base de lait.

19. Utilisation selon la revendication 18, dans laquelle la matière pâteuse est utilisée en une quantité dans la plage de 0,8 à 1,5% en masse, de préférence de 1,0 à 1,2% en masse.
